# EUROPÄISCHE PATENTSCHRIFT

(11) **EP 1 736 100 B1**
(45) Veröffentlichungstag und Bekanntmachung des Hinweises auf die Patenterteilung: **17.10.2012**
(21) Anmeldenummer: 06012002.9
(22) Anmeldetag: 10.06.2006
(51) Int. Cl.: A61B 5/151

(54) **Analysehandgerät zum Untersuchen von Körperflüssigkeiten**
Hand-held analysis device for examining body fluids
Appareil portable d'analyse des fluides corporels

(30) Priorität: 23.06.2005 EP 05013559
(43) Veröffentlichungstag der Anmeldung: 27.12.2006
(73) Patentinhaber: Roche Diagnostics GmbH, 68305 Mannheim (DE); F.Hoffmann-La Roche AG, 4070 Basel (CH)
(72) Erfinder: Kistner, Michael, 67071 Ludwigshafen (DE); Deck, Frank, 67150 Niederkirchen (DE)
(74) Vertreter: Petirsch, Markus

(56) Entgegenhaltungen:
- WO-A-98/24366
- WO-A-2005/006985
- US-A1- 2004 230 216
- US-A1- 2005 011 759
- US-E- R E32 922
- PATENT ABSTRACTS OF JAPAN Bd. 2000, Nr. 11, 3. Januar 2001 (2001-01-03) & JP 2000 217804 A (KDK CORP), 8. August 2000 (2000-08-08)

## Beschreibung

Die Erfindung betrifft ein Analysehandgerät zum Untersuchen von Körperflüssigkeit, umfassend ein Gehäuse mit einer Gehäuseöffnung, an die ein Körperteil, insbesondere ein Finger, zum Erzeugen einer Einstichwunde anlegbar ist, eine Analyseeinrichtung zum Untersuchen einer Körperflüssigkeit, die nach der Erzeugung einer Einstichwunde aus dieser gewonnen wurde, eine Stecheinheit, die eine Lanzette und einen Lanzettenantrieb zum Erzeugen einer Einstich und Rückführbewegung der Lanzette umfasst, eine Transporteinrichtung, mit der die Stecheinheit zwischen einer Arbeitsposition, in der mit der Lanzette eine Einstichwunde in einem an der Gehäuseöffnung anliegenden Körperteil erzeugbar ist, und einer Ruheposition beweglich ist, und eine Bedienungseinrichtung zum Betätigen eines Funktionsmechanismus der Stecheinheit. Ein derartiges Analysehandgerät ist aus der DE 10332488 A1 bekannt.

Je nach Tiefe der Einstichwunde handelt es sich bei der Körperflüssigkeit um interstitielle Flüssigkeit und/oder Blut. Nachfolgend wird ohne Beschränkung der Allgemeinheit auf Blut als Beispiel der zu untersuchenden Körperflüssigkeit Bezug genommen.

Mit Analysehandgeräten, die zusätzlich zu einer Analyseeinrichtung zum Untersuchen von Blut eine Stecheinheit zum Erzeugen einer Einstichwunde enthalten, wie z.B. in den Dokumenten JP 2000 217 804, WO 98/24366 und US R E32 922 offenbart, läßt sich eine Messung des Blutzuckerspiegels wesentlich einfacher durchführen als mit Analysesystemen, die aus einem Analysehandgerät, einem separaten Lanzettengerät und Teststreifen für die Blutuntersuchung bestehen.

Bei Analysesystemen mit separaten Geräten muß ein Benutzer zuerst mit einem Lanzettengerät eine Einstichwunde erzeugen, anschließend austretendes Blut auf einen Teststreifen aufbringen und diesen schließlich für die eigentliche Messung der Blutglucosekonzentration in ein Analysehandgerät einführen.

Mit integrierten Analysehandgeräten der eingangs genannten Art wird der Vorgang der Blutgewinnung und Messung für den Benutzer wesentlich vereinfacht. Es genügt, das Analysehandgerät mit seiner Gehäuseöffnung an einen Finger anzulegen. Mit der in das Gerät integrierten Stecheinheit wird eine Einstichwunde erzeugt. Anschließend austretendes Blut gelangt ohne weiteres Zutun des Benutzers zu der Analyseeinrichtung und wird von dieser untersucht. Dabei befindet sich die Stecheinheit zum Erzeugen der Einstichwunde zunächst in ihrer Arbeitsposition an der Gehäuseöffnung. Anschließend wird sie von einer Transporteinrichtung in eine Ruheposition bewegt, so daß die Gehäuseöffnung und damit die erzeugte Einstichwunde für eine Probenaufnahme zugänglich sind.

Da Diabetiker ihren Blutzuckerspiegel mehrmals täglich messen und deshalb entsprechende Analysehandgeräte ständig mit sich führen müssen, besteht die Forderung, Analysehandgeräte möglichst klein und kompakt zu fertigen. Wichtig in diesem Zusammenhang ist ein möglichst geringer Energieverbrauch, da die Leistung interner Stromquellen begrenzt ist. Häufige Batteriewechsel schränken den Benutzerkomfort ein. Größere Batterien sind mit der Forderung einer kompakten Bauweise des Analysehandgerätes nicht vereinbar.

Aufgabe der Erfindung ist es deshalb, einen Weg aufzuzeigen, wie ein Analysehandgerät der eingangs genannten Art kompakter gefertigt und ohne Einschränkung des Benutzerkomforts mit geringerem Energieaufwand betrieben werden kann.

Zur Lösung der Aufgabe wird von einem oben beschriebenen Analysehandgerät zum Untersuchen von Körperflüssigkeit ausgegangen, das ein Gehäuse, eine Analyseeinrichtung, eine Stecheinheit, eine Bedienungseinrichtung und eine Transporteinrichtung umfasst. Mit der Transporteinrichtung wird die Stecheinheit einschließlich des Lanzettenantriebs zwischen einer Arbeitsposition und einer Ruheposition bewegt. In der Arbeitsposition befindet sich die Stecheinheit derartig an der Gehäuseöffnung, dass mit der Lanzette eine Einstichwunde in einem an der Gehäuseöffnung anliegenden Körperteil erzeugt und eine Rückführbewegung ausgefuhrt werden kann. In der Ruheposition ist die Stecheinheit derart von der Gehäuseöffnung entfernt, dass der Raum vor der Gehäuseöffnung für die Analyseeinrichtung frei ist, so dass sie in eine Position für die Aufnahme von Blut, das aus dem an der Gehäuseöffnung anliegenden Körperteil austritt, bewegt werden kann.

Diese Aufgabe wird erfindungsgemäß dadurch gelöst, daß die Stecheinheit in einer ihrer beiden Positionen von der Bedienungseinrichtung entkoppelt ist und in der anderen Position mit der Bedienungseinrichtung gekoppelt ist, vorzugsweise mit ihr mechanisch in Wirkeingriff steht.

Die Stecheinheit eines Analysehandgerätes hat typischerweise mehrere Funktionsmechanismen, die beispielsweise dem Einstellen der Stechtiefe oder dem Spannen einer Antriebsfeder dienen. Für jeden dieser Funktionsmechanismen ist eine Bedienungseinrichtung erforderlich, damit der entsprechende Funktionsmechanismus betätigt werden kann. Die Bedienungseinrichtung weist ein entsprechendes Bedienungselement, beispielsweise einen Drehknopf oder eine Taste, für den Benutzer auf.

Bevorzugt ist die Stecheinheit in ihrer Ruheposition mit der Betätigungseinrichtung, beispielsweise zum Spannen einer Antriebsfeder oder dem Einstellen der Stechtiefe, gekoppelt und in ihrer Arbeitsposition von der Betätigungseinrichtung entkoppelt. Möglich ist es aber auch, daß die Stecheinheit in ihrer Ruheposition von der Bedienungseinrichtung entkoppelt ist und in der Arbeitsposition mit der Bedienungseinrichtung in Wirkeingriff steht.

Als Funktionsmechanismus werden unterschiedliche Mechanismen der Stecheinheit bezeichnet, insbesondere ein Spannmechanismus, der zum Spannen einer Antriebsfeder des Lanzettenantriebs dient, ein Stechtiefeneinstellmechanismus, mit dem die Stechtiefe der Lanzette in das Körperteil verändert und eingestellt werden kann, und ein Magazinbewegungsmechanismus, mit dem ein Lanzettenmagazin weitergeschaltet werden kann, so dass eine andere Lanzette mit dem Lanzettenantrieb in Eingriff kommen kann. Die Stecheinheit weist einen oder mehrere dieser Funktionsmechanismen auf. Die Bedienung durch die Bedienungseinrichtung kann insbesondere mittels einer Drehbewegung oder einer translatorischen Bewegung erfolgen. Der Funktionsmechanismus kann auch ein Auslösemechanismus sein, mit dem die vorgespannte Stecheinheit ausgelöst wird, so dass die Lanzette eine Einstichbewegung durchführt, um in das an der Gehäuseöffnung des Analysehandgeräts anliegende Körperteil einzustechen.

Mit der Erfindung läßt sich der Energiebedarf des Analysehandgerätes senken, da nur die Stecheinheit selbst und nicht zusätzlich auch noch die Bedienungseinrichtung zwischen der Ruheposition und der Arbeitsposition hin und her bewegt werden muß. Die zu bewegende Masse wird auf ein Minimum reduziert, so daß der Leistungsbedarf der Transporteinheit entsprechend gering ist. Als weiterer Vorteil kommt hinzu, daß bei einem erfindungsgemäßen Gerät die Gefahr von Fehlbedienungen reduziert ist, da der Funktionsmechanismus nur dann betätigt werden kann, wenn er mit der Bedienungseinrichtung gekoppelt ist. Ist der Funktionsmechanismus von der Bedienungseinrichtung entkoppelt, so hat eine fehlerhafte Betätigung der Bedienungseinrichtung keine Auswirkungen auf den Funktionsmechanismus.

Die erfindungsgemäße Entkopplung der Bedienungseinrichtung von der Stecheinheit ermöglicht ferner eine kompaktere Bauweise des Analysehandgerätes. Dies liegt daran, daß in dem Gehäuse ein relativ kleiner Freiraum ausreicht, in dem die Stecheinheit zwischen der Arbeitsposition und der Ruheposition bewegt werden kann und kein zusätzlicher Freiraum für eine entsprechende Bewegung der Bedienungseinrichtung erforderlich ist.

Bei der bisher gebräuchlichen permanenten Kopplung der Bedienungseinrichtung mit der Stecheinheit müssen mechanische Bedienungselemente, beispielsweise mittels Drehknöpfen bewegliche Wellen, durch Schlitze in der Gehäusewand hindurch geführt werden, damit sie zusammen mit der Stecheinheit beweglich sind. Derartige Schlitze haben den Nachteil, daß durch sie Schmutz in das Gehäuseinnere gelangen kann. Anstatt an dem bewährten Konzept einer permanenten Kopplung festzuhalten und nachteilige Schlitze beispielsweise durch elektrische Betätigungselemente, die über flexible Drähte mit beweglichen Teilen der Bedienungseinrichtung verbunden sind, zu vermeiden, geht die Erfindung mit einer Bedienungseinrichtung, die je nach Position der Stecheinheit mit ihr gekoppelt oder entkoppelt ist, einen neuen Weg. Auf diese Weise können mit einer relativ einfachen mechanischen Konstruktion die oben genannten Vorteile einer kompakteren Bauweise und einem geringen Stromverbrauch genutzt werden.

Die Aussage, daß die Stecheinheit von der Bedienungseinrichtung entkoppelt ist, ist im Rahmen der vorliegenden Anmeldung so zu verstehen, daß in diesem Zustand keine Kraftübertragung von der Bedienungseinrichtung auf den Funktionsmechanismus der Stecheinheit möglich ist. Bevorzugt ist in dem entkoppelten Zustand auch kein Kontakt zwischen der Bedienungseinrichtung und der Stecheinheit vorhanden. Eine Kopplung kann elektrisch, beispielsweise durch das Schließen eines elektrischen Kontakts erfolgen, geschieht jedoch bevorzugt dadurch, daß die Bedienungseinrichtung mit dem Funktionsmechanismus mechanisch in Wirkeingriff tritt. Steht die Bedienungseinrichtung mit der Stecheinheit in Wirkeingriff, so kann mechanisch eine Kraft von der Bedienungseinrichtung auf den Funktionsmechanismus der Stecheinheit übertragen werden. Bevorzugt wird zum Betätigen des Funktionsmechanismus über den Wirkeingriff eine Drehbewegung, beispielsweise über Wellen oder Zahnräder, von der Bedienungseinrichtung auf den Funktionsmechanismus übertragen. Dies ist durch eine kraftschlüssige oder - bevorzugt durch eine formschlüssige Kopplung möglich.

Weitere Einzelheiten und Vorteile der Erfindung werden im folgenden anhand eines Ausführungsbeispiels unter Bezugnahme auf die beigefügte Figuren näher erläutert. Die darin dargestellten Besonderheiten können einzeln oder in Kombination verwendet werden, um bevorzugte Ausgestaltungen zu schaffen. Es zeigen:
- Fig. 1: eine schematische Darstellung eines Analysehandgerätes bei aufgeschnittenem Gehäuse;
- Fig. 2: eine Detailansicht des in Figur 1 gezeigten Analysehandgerätes, in der sich die Stecheinheit in der Arbeitsposition befindet; und
- Fig. 3: eine Detailansicht der Stecheinheit in der Ruheposition.

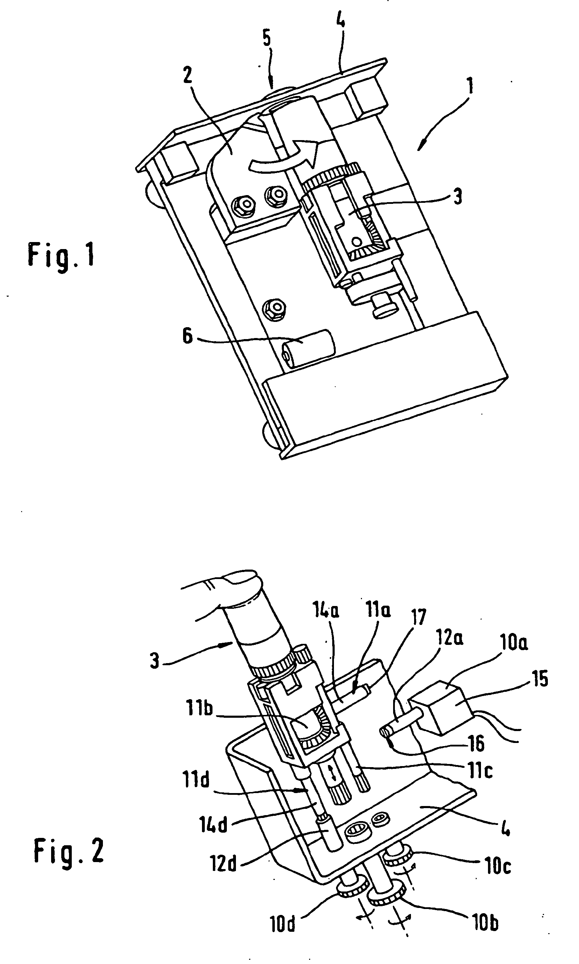

In Figur 1 ist schematisch ein Ausführungsbeispiel eines Analysehandgerätes 1 dargestellt, das eine Analyseeinrichtung 2 zur Untersuchung von Blut und eine Stecheinheit 3 zum Erzeugen einer Einstichwunde umfasst. Die Stecheinheit 3 umfasst eine Lanzette (nicht dargestellt) und einen Lanzettenantrieb (nicht dargestellt) zum Erzeugen einer Einstich- und Rückführbewegung der Lanzette. Das Analysehandgerät 1 hat ein Gehäuse 4 mit einer Gehäuseöffnung 5, an die ein Körperteil, beispielsweise ein Finger zum Erzeugen einer Einstichwunde angelegt wird.

Befindet sich die Stecheinheit 3 in der in Figur 1 dargestellten Arbeitsposition, so kann eine Einstichwunde in einem an der Gehäuseöffnung 5 anliegenden Körperteil erzeugt werden. Nach einem Einstich wird die Stecheinheit 3 mittels einer Transporteinrichtung (nicht dargestellt) durch eine Schwenkbewegung oder durch eine translatorische Schiebebewegung in eine Ruheposition befördert. Die Analyseeinrichtung 2 wird in den dabei freiwerdenden Raum zu der Gehäuseöffnung 5 bewegt, so daß die Analyseeinrichtung 2 in eine Position für die Aufnahme von Blut gelangt, das aus der erzeugten Einstichwunde ausgetreten ist.

Bei dem dargestellten Ausführungsbeispiel enthält die Analyseeinrichtung 2 eine Kassette mit einem als Band ausgeführten Teststreifen. Der Teststreifen weist eine Vielzahl von Bandabschnitten auf, die mit Chemikalien beschichtet sind, die mit aufgebrachtem Blut reagieren und dabei eine der Blutzuckerkonzentration entsprechende, optisch detektierbare Farbänderung bewirken. Die Analyseeinrichtung 2 und sonstige elektrischen Baueinheiten des Analysehandgeräts 1 werden von einer internen Stromquelle 6 in Form einer handelsüblichen Batterie mit Energie versorgt.

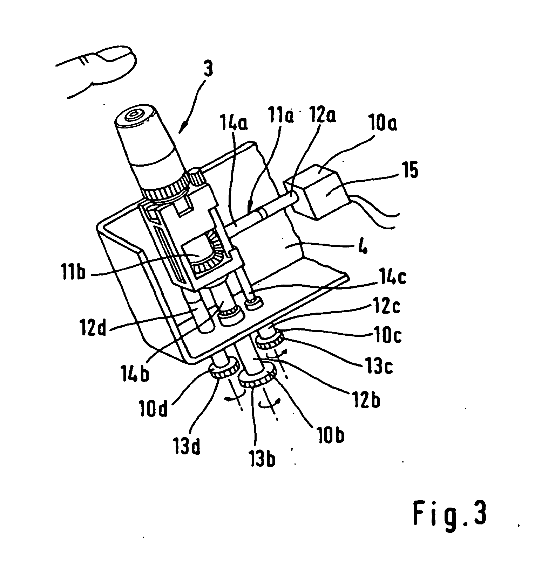

In Figur 1 sind der besseren Übersichtlichkeit wegen keine Bedienungseinrichtungen zum Betätigen von Funktionsmechanismen der Stecheinheit 3 dargestellt. Diese Bedienungseinrichtungen und Funktionsmechanismen der Stecheinheit 3 werden im Folgenden anhand der Figuren 2 und 3 erläutert.

In Figur 2 ist die Stecheinheit 3 ebenfalls in der Arbeitsposition, wie in Figur 1, dargestellt. Figur 3 zeigt dagegen die Ruheposition der Stecheinheit 3. Die Stecheinheit 3 wechselt zwischen diesen beiden Positionen hin und her. Die Analyseeinrichtung 2 wird von ihrer in Figur 1 dargestellten von der Gehäuseöffnung 5 entfernten Position in eine Analyseposition bewegt, in der die Analyseeinrichtung 2 so weit an die Gehäuseöffnung 5 angenähert ist, dass eine Analyse einer Flüssigkeit möglich ist, die aus einer Wunde eines Körperteils austritt, das an der Gehäuseöffnung 5 positioniert ist. Dabei nimmt die Analyseeinrichtung 2 im wesentlichen die Position an der Gehäuseöffnung ein, die die Stecheinrichtung 3 in ihrer Arbeitsposition einnimmt. Die Analyseeinrichtung 2 und die Stecheinrichtung 3 werden also alternierend in unmittelbare Nähe der Gehäuseöffnung 5 bewegt.

In Figur 2 ist die Stecheinheit 3 in der Arbeitsposition zusammen mit verschiedenen Bedienungseinrichtungen 10a,10b,10c,10d dargestellt, die zum Betätigen von Funktionsmechanismen 11a,11b,11c,11d der Stecheinheit 3 dienen. Bei dem dargestellten Ausführungsbeispiel hat die Stecheinheit 3 einen ersten Funktionsmechanismus 11a zum Spannen einer Antriebsfeder, einen zweiten Funktionsmechanismus 11 c zum Einstellen der Stechtiefe, einen dritten Funktionsmechanismus 11 b zum Drehen eines Lanzettenmagazins, das in der Stecheinheit 3 gelagert ist, und einen vierten Funktionsmechanismus 11d zum Auslösen einer Einstich- und Rückführbewegung einer Lanzette.

Eine Stecheinheit mit derartigen Funktionsmechanismen ist im Stand der Technik bekannt und beispielsweise in der deutschen Patentanmeldung 102004059491.0 beschrieben, die diesbezüglich durch Bezugnahme zum Gegenstand der Anmeldung gemacht wird.

In der in Figur 2 dargestellten Arbeitsposition der Stecheinheit 3 steht nur die Bedienungseinrichtung 10d mit der Stecheinheit 3, genauer gesagt dem Funktionsmechanismus 11d, in Wirkeingriff. Die übrigen Bedienungseinrichtungen 10a,10b,10c sind von der Stecheinheit 3, genauer gesagt den dazugehörenden Funktionsmechanismen 11 a, 11b bzw. 11 c, entkoppelt. In Figur 3 ist die Stecheinheit 3 in ihrer Ruheposition dargestellt. In der Ruheposition stehen die Bedienungseinrichtungen 10a,10b und 10c mit der Stecheinheit 3 in Wirkeingriff, während die Bedienungseinrichtung 10d von der Stecheinheit 3 entkoppelt ist.

Der Wirkeingriff der Stecheinheit 3 mit den Bedienungseinrichtungen 10a,10b,10c,10d dient dem Übertragen von Drehbewegungen von der jeweiligen Bedienungseinrichtung 10a, 10b, 10c, 10d auf den dazugehörenden Funktionsmechanismus 11a,11b,11c,11d der Stecheinheit 3. Jede Bedienungseinrichtung 10a,10b,10c,10d umfasst zu diesem Zweck eine Welle 12a,12b,12c bzw. 12d, die formschlüssig mit dem jeweiligen Funktionsmechanismus 11a,11b,11c bzw. 11 d der Stecheinheit 3 gekoppelt werden kann.

Zu der Bedienungseinrichtung 10d gehört eine durch die Außenwand des Gehäuses 4 hindurchragende. Welle 12d, die an ihren aus dem Gehäuse 4 herausragenden Ende einen Drehknopf 13d trägt. Die Welle 12d ist als Hohlwelle ausgeführt, so daß sie auf eine passende Welle 14d (Figur 2) des Funktionsmechanismus 11 d, mit dem die Stecheinheit 3 ausgelöst werden kann, aufgeschoben werden kann. Die beiden Wellen 12d und 14d sind in der in Figur 2 dargestellten Arbeitsposition der Stecheinheit 3 über eine Keilwellenverbindung verbunden. Dabei sind die Keilnaben nur in einem vorderen Abschnitt der Welle 12d vorhanden. Der Innendurchmesser der Hohlwelle 12d nimmt hinter diesem Abschnitt mit den Keilnaben etwas zu, so daß in der in Figur 3 dargestellten Ruheposition der Stecheinheit 3 keine Drehbewegung von der Welle 12d auf die Welle 14d übertragen werden kann. Die Bedienungseinrichtung 10d also von dem Funktionsmechanismus 11 d entkoppelt ist.

Der Aufbau der Bedienungseinrichtungen 10b und 10c entspricht dem Aufbau der Bedienungseinrichtung 10d. Die Bedienungseinrichtungen 10b und 10c umfassen jeweils eine durch eine Außenwand des Gehäuses 4 hindurchragende Welle 12b bzw. 12c, an deren Ende ein für ein Benutzer zugänglicher Drehknopf 13b bzw. 13c angebracht ist. Die Wellen 12b und 12c treten mit entsprechenden Wellen 14b bzw. 14c der Funktionsmechanismen 11 b bzw. 11 c über eine Keilwellenverbindung in Wirkeingriff, sobald sich die Stecheinheit 3 in der in Figur 3 dargestellten Ruheposition befindet. Anstelle einer Keilwellenverbindung können zur Kopplung der Wellen 12b und 14b bzw. 12c und 14c auch andere formschlüssige Verbindungen, beispielsweise Stirnverzahnungen oder Polygonprofile verwendet werden.

Die Bedienungseinrichtung 10a, mit welcher der Funktionsmechanismus 11a zum Spannen einer Antriebsfeder der Stecheinheit 3 betätigt wird, umfasst einen Elektromotor 15, von dem eine Welle 12a angetrieben wird, die in der in Figur 3 dargestellten Ruheposition der Stecheinheit 3 mit der Welle 14a des Funktionsmechanismus 11a gekoppelt ist. Die Bedienungseinrichtung 10a umfasst ferner ein Bedienungselement (nicht dargestellt, beispielsweise in Form einer Taste, mit der ein Benutzer den Elektromotor einschalten kann.

Im Unterschied zu den Wellen 12b,12c und 12d verläuft die Welle 12a quer zu der Richtung, in der die Stecheinheit 3 bei ihrer Bewegung von der Ruheposition in die Arbeitsposition verschoben wird. Die Welle 12a weist deshalb an ihrem von dem Motor 15 abgewandten Ende einen Schlitz 16 auf, in den ein Steg 17 an dem freien Ende der Welle 14a nach dem Prinzip einer Nut- und Federverbindung eingreift, wenn sich die Stecheinheit 3 in der in Figur 3 dargestellten Ruheposition befindet.

## Patentansprüche

1. Analysehandgerät zum Untersuchen von Körperflüssigkeit, umfassend:
ein Gehäuse (4) mit einer Gehäuseöffnung (5), an die ein Körperteil, insbesondere ein Finger, zum Erzeugen einer Einstichwunde anlegbar ist,
eine Analyseeinrichtung (2) zum Untersuchen einer an der Einstichwunde gewonnenen Probe einer Körperflüssigkeit,
eine Stecheinheit (3), die eine Lanzette und einen Lanzettenantrieb zum Erzeugen einer Einstich- und Rückführbewegung der Lanzette einschließt,
eine Transporteinrichtung, mit der die Stecheinheit (3) einschließlich des Lanzettenantriebs zwischen einer Arbeitsposition und einer Ruheposition beweglich ist, wobei sich die Stecheinheit (3) in der Arbeitsposition derartig an der Gehäuseöffnung (5) befindet, dass die Lanzette eine Einstich- und Rückführbewegung durchführt und mit ihr eine Einstichwunde in einem an der Gehäuseöffnung (5) anliegenden Körperteil erzeugt werden kann, und die Stecheinheit (3) in der Ruheposition derartig von der Gehäuseöffnung (5) entfernt ist, dass der Raum vor der Gehäuseöffnung (5) für die Analyseeinrichtung (2) frei ist, so dass sie in eine Position für die Aufnahme von Blut bewegt werden kann, das aus dem an der Gehäuseöffnung (5) anliegenden Körperteil austritt, und
eine Bedienungseinrichtung (10a,10b,10c,10d) zum Betätigen eines Funktionsmechanismus (11a,11b,11c,11d) der Stecheinheit (3),
wobei die Stecheinheit (3) in einer ihrer beiden Positionen von der Bedienungseinrichtung (10a,10b,10c,10d) entkoppelt ist und in der anderen Position mit der Bedienungseinrichtung (10a,10b,10c,10d) gekoppelt ist, vorzugsweise mechanisch mit ihr in Wirkeingriff steht.

2. Analysehandgerät nach Anspruch 1, **dadurch gekennzeichnet, dass**
in der Position, in der die Bedienungseinrichtung (10a,10b,10c,10d) von der Stecheinheit (3) entkoppelt ist, keine Kraftübertragung von der Bedienungseinrichtung (10a,10b,10c,10d) auf den Funktionsmechanismus (11a,11b,11c,11d) der Stecheinheit (3) möglich ist, und
in der anderen Position, in der die Bedienungseinrichtung (10a,10b,10c,10d) mit der Stecheinheit (3) gekoppelt ist, eine Kraft von der Bedienungseinrichtung (10a,10b,10c,10d) auf den Funktionsmechanismus (11a,11b,11c,11d) übertragen werden kann.

3. Analysehandgerät nach einem der vorhergehenden Ansprüche, **dadurch gekennzeichnet, dass** die Stecheinheit (3) durch eine Schwenkbewegung und/oder eine translatorische Bewegung zwischen ihrer Ruheposition und ihrer Arbeitsposition bewegt wird.

4. Analysehandgerät nach einem der vorhergehenden Ansprüche, **dadurch gekennzeichnet, dass** die Analyseeinrichtung (2) eine Kassette mit einem bandförmigen Teststreifen umfasst.

5. Analysehandgerät nach einem der vorhergehenden Ansprüche, **dadurch gekennzeichnet, dass** der Funktionsmechanismus (11a,11b,11c,11d) ausgewählt ist aus der Gruppe bestehend aus einem Spannmechanismus zum Spannen einer Antriebsfeder, einem Stechtiefeneinstellmechanismus, einem Magazinbewegmechanismus und einem Auslösemechanismus zum Auslösen einer Stichbewegung einer Lanzette.

6. Analysehandgerät nach einem der vorhergehenden Ansprüche, **dadurch gekennzeichnet, dass** die Stecheinheit (3) in der Arbeitsposition von der Bedienungseinrichtung (10a,10b,10c) entkoppelt ist und in der Ruheposition mit der Bedienungseinrichtung (10a,10b,10c) in Wirkeingriff steht.

7. Analysehandgerät nach einem der vorhergehenden Ansprüche, **dadurch gekennzeichnet, dass** der Wirkeingriff der Stecheinheit (3) mit der Bedienungseinrichtung (10a,10b,10c,10d) dem Übertragen einer Drehbewegung von der Bedienungseinrichtung (10a,10b,10c,10d) auf den Funktionsmechanismus (11a,11b,11c,11d) der Stecheinheit (3) dient.

8. Analysehandgerät nach einem der vorhergehenden Ansprüche, **dadurch gekennzeichnet, dass** die Bedienungseinrichtung (10a,10b,10c,10d) eine Welle (12a,12b,12c,12d) umfasst.

9. Analysehandgerät nach Anspruch 8, **dadurch gekennzeichnet, dass** die Welle (12b,12c,12d) durch eine Außenwand des Gehäuses (4) hindurchragt.

10. Analysehandgerät nach einem der vorhergehenden Ansprüche, **dadurch gekennzeichnet, dass** der Wirkeingriff der Stecheinheit (3) mit der Bedienungseinrichtung (10a,10b,10c,10d) formschlüssig ist.

11. Analysehandgerät nach einem der vorhergehenden Ansprüche, **dadurch gekennzeichnet, dass** die Stecheinheit (3) einen durch eine Antriebsfeder antreibbaren Antriebsrotor umfasst.

12. Analysehandgerät nach Anspruch 11, **dadurch gekennzeichnet, dass** die Bedienungseinrichtung (10a) einen in dem Gehäuse (4) angeordneten Motor (15) zum Spannen der Antriebsfeder umfasst.

13. Analysehandgerät nach einem der vorhergehenden Ansprüche, **dadurch gekennzeichnet, dass** die Stecheinheit (3) eine Aufnahme für ein Lanzettenmagazin mit mehreren Lanzetten umfasst, die durch Drehen des Lanzettenmagazins nacheinander in eine Kopplungsposition beweglich sind, in der sie mit dem Lanzettenantrieb koppelbar sind, wobei der durch die Bedienungseinrichtung (10b) betätigbare Funktionsmechanismus (11b) der Stecheinheit (3) zum Drehen des Lanzettenmagazins dient.

14. Analysehandgerät nach einem der vorhergehenden Ansprüche, **dadurch gekennzeichnet, dass** es mehrere Bedienungseinrichtungen (10a,10b,10c,10d) aufweist, mit denen die Stecheinheit (3) in einer ihrer beiden Positionen in Wirkeingriff steht und von denen die Stecheinheit (3) in der anderen Position entkoppelt ist.

## Claims

1. Hand-held analysis instrument for analyzing a body fluid, comprising:
a housing (4) having a housing opening (5), to which a body part, in particular a finger, can be applied to generate a puncture wound,
an analysis unit (2) for analyzing a sample of a body fluid obtained at the puncture wound,
a piercing unit (3) including a lancet and a lancet drive for driving a puncture and retraction movement of the lancet,
a transport unit for moving the piercing unit (3), including the lancet drive, between an operating position and a rest position, the piercing unit (3) in the operating position being located at the housing opening (5) in such a manner that the lancet performs a puncture and retraction movement and that a puncture wound can be generated by the lancet in a body part pressed against the housing opening (5), and the piercing unit (3) is remote from the housing opening (5) in the rest position in such a manner that the space in front of the housing opening (5) is free for the analysis unit (2), so that the analysis unit (2) can be moved into a position for receiving blood coming out of the body part pressed against the housing opening (5), and
an operating device (10a, 10b, 10c, 10d) for actuating a functional mechanism (11a, 11b, 11c, 11d) of the piercing unit (3),
wherein the piercing unit (3) is decoupled from the operating device (10a, 10b, 10c, 10d) in one of its two positions and is coupled to the operating device (10a, 10b, 10c, 10d), preferably in mechanical engagement therewith in the other position.

2. Hand-held analysis instrument according to Claim 1, **characterized in that**
in the position in which the operating device (10a, 10b, 10c, 10d) is decoupled from the piercing unit (3), no force transmission is possible from the operating device (10a, 10b, 10c, 10d) to the functional mechanism (11a, 11b, 11c, 11d) of the piercing unit (3), and
in the other position, in which the operating device (10a, 10b, 10c, 10d) is coupled to the piercing unit (3), a force can be transmitted from the operating device (10a, 10b, 10c, 10d) to the functional mechanism (11a, 11b, 11c, 11d).

3. Hand-held analysis instrument according to any one of the preceding claims, **characterized in that** the piercing unit (3) is moved between its rest position and its operating position by a pivot movement and/or a translational movement.

4. Hand-held analysis instrument according to any one of the preceding claims, **characterized in that** the analysis unit (2) comprises a cassette including a band-shaped test strip.

5. Hand-held analysis instrument according to any one of the preceding claims, **characterized in that** the functional mechanism (11a, 11b, 11c, 11d) is selected from the group comprising a tensioning mechanism for tensioning a drive spring, a piercing depth setting mechanism, a position changing mechanism for a magazine, and a trigger mechanism for triggering a puncture movement of a lancet.

6. Hand-held analysis instrument according to any one of the preceding claims, **characterized in that** the piercing unit (3) is decoupled from the operating device (10a, 10b, 10c) in the operating position and is operatively engaged with the operating device (10a, 10b, 10c) in the rest position.

7. Hand-held analysis instrument according to any one of the preceding claims, **characterized in that** the operational engagement of the piercing unit (3) with the operating device (10a, 10b, 10c, 10d) is used for transmitting a rotational movement from the operating device (10a, 10b, 10c, 10d) to the functional mechanism (11a, 11b, 11c, 11d) of the piercing unit (3).

8. Hand-held analysis instrument according to any one of the preceding claims, **characterized in that** the operating device (10a, 10b, 10c, 10d) comprises a shaft (12a, 12b, 12c, 12d).

9. Hand-held analysis instrument according to Claim 8, **characterized in that** the shaft (12b, 12c, 12d) projects through an outer wall of the housing (4).

10. Hand-held analysis instrument according to any one of the preceding claims, **characterized in that** the operational engagement of the piercing unit (3) with the operating device (10a, 10b, 10c, 10d) is formfitting.

11. Hand-held analysis instrument according to any one of the preceding claims, **characterized in that** the piercing unit (3) comprises a drive rotor driven by a drive spring.

12. Hand-held analysis instrument according to Claim 11, **characterized in that** the operating device (10a) comprises a motor (15), located in the housing (4), for tensioning the drive spring.

13. Hand-held analysis instrument according to any one of the preceding claims, **characterized in that** the piercing unit (3) comprises a receptacle for a lancet magazine including a plurality of lancets which are movable in sequence into a coupling position, in which they can be coupled to the lancet drive, by rotating the lancet magazine, the functional mechanism (11b) of the piercing unit (3) being actuatable by the operating device (10b) and being used for rotating the lancet magazine.

14. Hand-held analysis instrument according to any one of the preceding claims, **characterized in that** it has a plurality of operating devices (10a, 10b, 10c, 10d), with which the piercing unit (3) is operatively engaged in one of its two positions and from which the piercing unit (3) is decoupled in the other position.

## Revendications

1. Appareil d'analyse portatif pour analyser un fluide corporel, comprenant :
un boîtier (4) comportant une ouverture de boîtier (5) contre laquelle peut s'appliquer une partie du corps, en particulier un doigt, pour former une incision,
un dispositif d'analyse (2) pour analyser un échantillon de fluide corporel obtenu sur l'incision,
un dispositif de piqûre (3) comprenant une lancette et un actionneur de lancette pour produire un mouvement de piqûre et de recul de la lancette,
un dispositif de transport permettant de déplacer le dispositif de piqûre (3), y compris l'actionneur de lancette, entre une position de travail et une position de repos, en position de travail le dispositif de piqûre (3) se trouvant sur l'ouverture de boîtier (5) de sorte que la lancette réalise un mouvement de piqûre et de recul et permet de former une incision dans une partie du corps appliquée contre ladite ouverture (5), et en position de repos le dispositif de piqûre (3) étant éloigné de l'ouverture de boîtier (5) de sorte que l'espace devant ladite ouverture (5) est libre pour le dispositif d'analyse (2), lequel peut ainsi passer dans une position permettant de recueillir le sang sortant de la partie du corps appliquée contre l'ouverture de boîtier (5), et
un dispositif de commande (10a, 10b, 10c, 10d) pour actionner un mécanisme de fonctionnement (11a, 11b, 11c, 11d) du dispositif de piqûre (3),
dans l'une de ses deux positions, le dispositif de piqûre (3) est découplé du dispositif de commande (10a, 10b, 10c, 10d) et dans l'autre position, il est couplé au dispositif de commande (10a, 10b, 10c, 10d) et de préférence en prise active mécanique avec celui-ci.

2. Appareil d'analyse portatif selon la revendication 1, **caractérisé en ce que**
dans la position dans laquelle le dispositif de commande (10a, 10b, 10c, 10d) est découplé du dispositif de piqûre (3), une transmission de force du dispositif de commande (10a, 10b, 10c, 10d) au mécanisme de fonctionnement (11a, 11b, 11c, 11d) du dispositif de piqûre (3), n'est pas possible, et
dans l'autre position dans laquelle le dispositif de commande (10a, 10b, 10c, 10d) est couplé au dispositif de piqûre (3), il est possible de transmettre une force du dispositif de commande (10a, 10b, 10c, 10d) au mécanisme de fonctionnement (11a, 11b, 11c, 11d).

3. Appareil d'analyse portatif selon l'une des revendications précédentes, **caractérisé en ce que** le dispositif de piqûre (3) est déplacé par un mouvement pivotant et/ou un mouvement translatoire entre sa position de repos et sa position de travail.

4. Appareil d'analyse portatif selon l'une des revendications précédentes, **caractérisé en ce que** le dispositif d'analyse (2) comprend une cassette contenant une bandelette de test en forme de bande.

5. Appareil d'analyse portatif selon l'une des revendications précédentes, **caractérisé en ce que** le mécanisme de fonctionnement (11a, 11b, 11c, 11d) est choisi parmi le groupe constitué par un mécanisme tendeur permettant de tendre un ressort de commande, un mécanisme de réglage de la profondeur de piqûre, un mécanisme de déplacement de cartouche et un mécanisme de déclenchement permettant de déclencher un mouvement de piqûre d'une lancette.

6. Appareil d'analyse portatif selon l'une des revendications précédentes, **caractérisé en ce que** le dispositif de piqûre (3) est découplé du dispositif de commande (10a, 10b, 10c) en position de travail et en prise active avec le dispositif de commande (10a, 10b, 10c) en position de repos.

7. Appareil d'analyse portatif selon l'une des revendications précédentes, **caractérisé en ce que** la prise active du dispositif de piqûre (3) avec le dispositif de commande (10a, 10b, 10c, 10d) sert à transmettre un mouvement de rotation du dispositif de commande (10a, 10b, 10c, 10d) au mécanisme de fonctionnement (11a, 11b, 11c, 11d) du dispositif de piqûre (3).

8. Appareil d'analyse portatif selon l'une des revendications précédentes, **caractérisé en ce que** le dispositif de commande (10a, 10b, 10c, 10d) comprend un arbre (12a, 12b, 12c, 12d).

9. Appareil d'analyse portatif selon la revendication 8, **caractérisé en ce que** l'arbre (12b, 12c, 12d) traverse une paroi extérieure du boîtier (4).

10. Appareil d'analyse portatif selon l'une des revendications précédentes, **caractérisé en ce que** la prise active du dispositif de piqûre (3) avec le dispositif de commande (10a, 10b, 10c, 10d) est une prise par complémentarité de forme.

11. Appareil d'analyse portatif selon l'une des revendications précédentes, **caractérisé en ce que** le dispositif de piqûre (3) comprend un rotor de commande actionnable par un ressort de commande.

12. Appareil d'analyse portatif selon la revendication 11, **caractérisé en ce que** le dispositif de commande (10a) comprend un moteur (15) logé dans le boîtier (4), permettant de tendre le ressort de commande.

13. Appareil d'analyse portatif selon l'une des revendications précédentes, **caractérisé en ce que** le dispositif de piqûre (3) comprend un logement pour une cartouche de lancettes contenant plusieurs lancettes, lesquelles peuvent passer successivement, par rotation de la cartouche de lancettes, dans une position de couplage dans laquelle elles peuvent être couplées à l'actionneur de lancette, le mécanisme de fonctionnement (11 b) du dispositif de piqûre (3), pouvant être actionné par le dispositif de commande (10b), servant à la rotation de la cartouche de lancettes.

14. Appareil d'analyse portatif selon l'une des revendications précédentes, **caractérisé en ce qu'**il présente plusieurs dispositifs de commande (10a, 10b, 10c, 10d) avec lesquels le dispositif de piqûre (3) est en prise active dans l'une de ses deux positions et desquels le dispositif de piqûre (3) est découplé dans l'autre position.
